(19) Europäisches Patentamt
European Patent Office
Office européen des brevets

(11) EP 4 234 558 A1

(12) EUROPEAN PATENT APPLICATION
published in accordance with Art. 153(4) EPC

(43) Date of publication:
**30.08.2023 Bulletin 2023/35**

(21) Application number: **21902605.1**

(22) Date of filing: **07.12.2021**

(51) International Patent Classification (IPC):
***C07D 487/04*** *(2006.01)* ***C07D 471/04*** *(2006.01)*
***A61K 31/437*** *(2006.01)* ***A61P 35/00*** *(2006.01)*
***A61P 35/02*** *(2006.01)*

(52) Cooperative Patent Classification (CPC):
**A61K 31/437; A61P 35/00; A61P 35/02; C07D 471/04; C07D 487/04**

(86) International application number:
**PCT/CN2021/136117**

(87) International publication number:
**WO 2022/121900 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.12.2020 CN 202011437580**

(71) Applicant: **Zhejiang Longcharm Bio-Tech Pharma. Co., Ltd.**
**Hangzhou, Zhejiang, 310018 (CN)**

(72) Inventors:
- **ZHANG, Yang**
  **Shanghai 200131 (CN)**
- **WU, Wentao**
  **Shanghai 200131 (CN)**
- **GENG, Kaijun**
  **Shanghai 200131 (CN)**
- **XU, Yangyang**
  **Shanghai 200131 (CN)**
- **CHEN, Shuhui**
  **Shanghai 200131 (CN)**

(74) Representative: **Icosa**
**83 avenue Denfert-Rochereau**
**75014 Paris (FR)**

# (54) PYRROLOPYRIDINE COMPOUND AND APPLICATION THEREOF

(57) A pyrrolopyridine compound and an application thereof. Specifically disclosed is a compound represented by formula (P) or a pharmaceutically acceptable salt thereof.

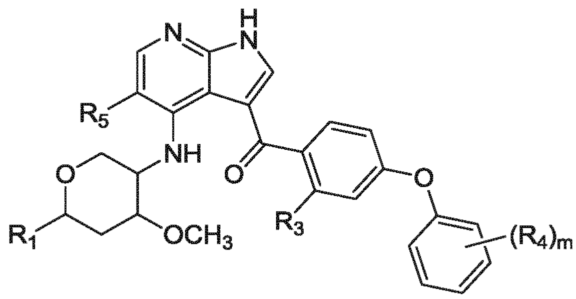

(P)

EP 4 234 558 A1

## Description

The present disclosure claims the priority to: Chinese Patent Application No. CN202011437580.2 filed on December 7, 2020.

## Technical Field

The present disclosure relates to a pyrrolopyridine compound, in particular to a compound of formula (P) or a pharmaceutically acceptable salt thereof.

## Background Art

The pyrrolopyridine compounds of the present invention are a new class of protein kinase inhibitors with various therapeutic applications, which can be used to treat the proliferation, inflammation, autoimmune and other related disorders caused by protein kinase.

Kinases are enzymes that catalyze the transfer of phosphate groups from phosphate donors (such as ATP) to specific substrates. Protein kinases are a large subset of kinases that play key roles in regulating various cell signals and processes, among which one is called BTK.

BTK is a member of the TEC family of cytoplasmic tyrosine kinases (TFKs). TFKs consists of five members, in addition to BTK, namely ITK, TEC, BMX and TXK. TFKs have an evolutionary history of over 600 million years and belong to a very ancient kinase family that mainly functions in the hematopoietic system.

BTK is mainly responsible for the transmission and amplification of various intracellular and extracellular signals in B lymphocytes, and is essential for normal B cell maturation. Inactivation of BTK function in XLA patients results in a deficiency of peripheral B cell and immunoglobulin. Signaling receptors upstream of BTK include growth factor and cytokine receptors, G protein-coupled receptors such as chemokine receptors, antigen receptors (especially B cell receptors [BCR]), and integrins, etc. BTK in turn activates many major downstream signaling pathways, including phosphoinositide-3 kinase (PI3K)-AKT pathway, phospholipase-C (PLC), protein kinase C, and nuclear factor κ B (NF-κB), etc. The role of BTK in BCR signaling and cell migration is well established, and these functions also appear to be major targets of BTK inhibitor. Increased BTK activity has been detected in blood cancer cells such as B-cell chronic lymphocytic leukemia (CLL) and mantle cell lymphoma (MCL), etc. Abnormal activity of BTK function frequently leads to B-cell malignancies or autoimmune diseases, making it a popular target for research and development.

Currently, BTK inhibitors approved by FDA for marketing include ibrutinib and acalabrutinib (acalanib). These two irreversible covalent inhibitors can covalently bind with protein C481, thereby effectively inhibiting the activity of BTK. Clinical studies have found that patients can develop resistance mutations after administration of ibrutinib and acalabrutinib (acalanib), including C481S, C481Y, C481R, C481F. For drug resistance caused by protein cysteine C481 mutation, we have designed and synthesized a series of irreversible BTK inhibitors of pyrrolopyridine.

## Summary of the Invention

The present disclosure provides a compound represented by formula (P) or a pharmaceutically acceptable salt thereof,

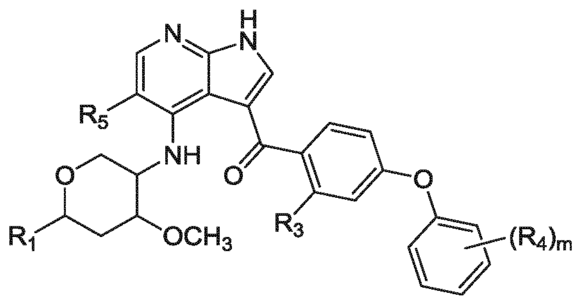

(P)

wherein,

$R_1$ is selected from CN and $C_{1-3}$ alkyl, wherein the $C_{1-3}$ alkyl is optionally substituted by 1, 2 or 3 $R_a$;
$R_3$ is selected from H, F, Cl, Br, I and $CH_3$;

$R_4$ is selected from H, F, Cl, Br, I and $CH_3$;
$R_5$ is selected from H, F, Cl, Br, I, CN, $CH_3$ and $OCH_3$;
m is selected from 1 and 2;
$R_a$ is selected from H, F, Cl, Br and OH.

The present disclosure provides a compound represented by formula (P) or a pharmaceutically acceptable salt thereof,

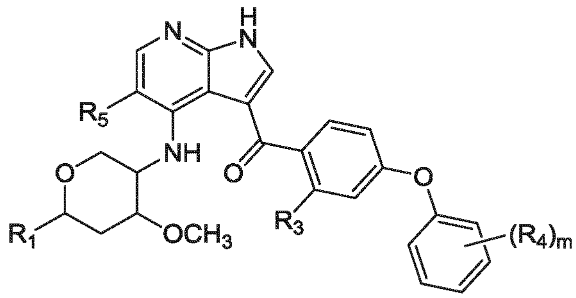

(P)

wherein,

$R_1$ is selected from CN and $C_{1-3}$ alkyl, wherein the $C_{1-3}$ alkyl is optionally substituted by 1, 2 or 3 $R_a$;
$R_3$ is selected from H, F, Cl, Br and I;
R4 is selected from H, F, Cl, Br and I;
$R_5$ is selected from CN and F;
m is selected from 1 and 2;
$R_a$ is selected from H, F, Cl, Br and OH.

In some embodiments of the present disclosure, the above-mentioned compound or pharmaceutically acceptable salt thereof is

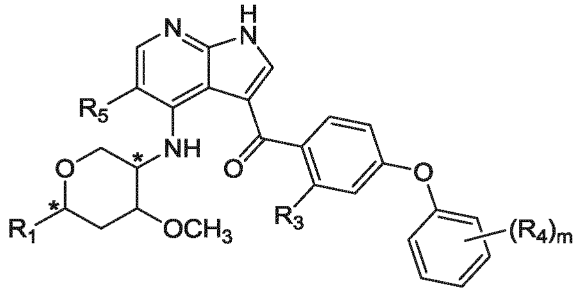

(P-1)

Wherein $R_1$, $R_3$, $R_4$, $R_5$ and m are as defined herein.

The carbon atoms with "*" are chiral carbon atoms and exist in the form of (*R*) or (*S*) single enantiomer or enriched in one enantiomer.

In some embodiments of the present disclosure, the above-mentioned $R_1$ is selected from CN, $CH_3$, $CH_2CH_3$ and CH $(CH_3)_2$, wherein the $CH_3$, $CH_2CH_3$ and CH $(CH_3)_2$ are optionally substituted by 1, 2 or 3 $R_a$. and other variables are as defined herein. In some embodiments of the present disclosure, the above-mentioned $R_1$ is selected from CN and $CH_2OH$, and other variables are as defined herein.

In some embodiments of the present disclosure, the above-mentioned $R_3$ is Cl, and other variables are as defined herein.

In some embodiments of the present disclosure, the above-mentioned $R_4$ is selected from H and F, and other variables are as defined herein.

In some embodiments of the present disclosure, the structural unit

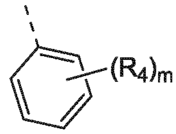

is

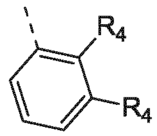

and other variables are as defined herein. In some embodiments of the present disclosure, The structural unit

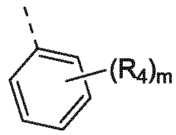

is selected from

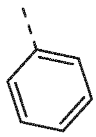

, 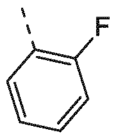 and 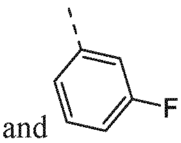,

and other variables as defined herein.

In some embodiments of the present disclosure, the above-mentioned compound or pharmaceutically acceptable salt thereof is

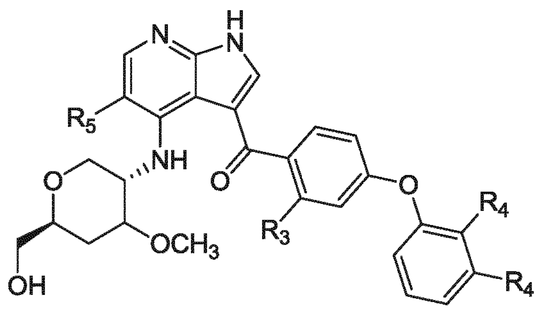

( P-2 )

wherein, $R_3$, $R_4$ and $R_5$ are as defined herein.

The present disclosure provides a compound shown in formula (I) or a pharmaceutically acceptable salt thereof,

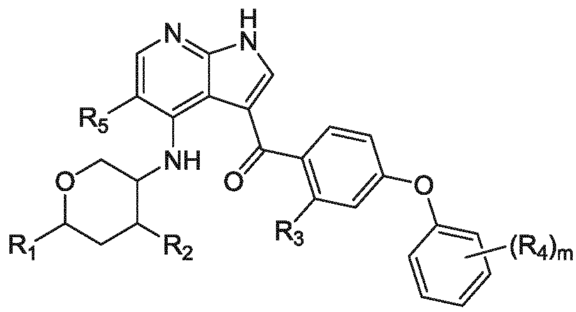

(I)

wherein, $R_1$ is selected from CN and $C_{1-3}$ alkyl, wherein the $C_{1-3}$ alkyl is optionally substituted by 1, 2 or 3 $R_a$;
$R_2$ is selected from H and $C_{1-3}$ alkoxy, wherein the $C_{1-3}$ alkoxy is optionally substituted by 1, 2 or 3 $R_b$;
$R_3$ is selected from H, F, Cl, Br and I;
$R_4$ is selected from H, F, Cl, Br and I;
$R_5$ is selected from CN and F;
m is selected from 1 and 2;
$R_a$ is selected from H, F, Cl, Br and OH;
$R_b$ is selected from H, F, Cl and Br.

In some embodiments of the present disclosure, the above-mentioned compound or pharmaceutically acceptable salt thereof is selected from,

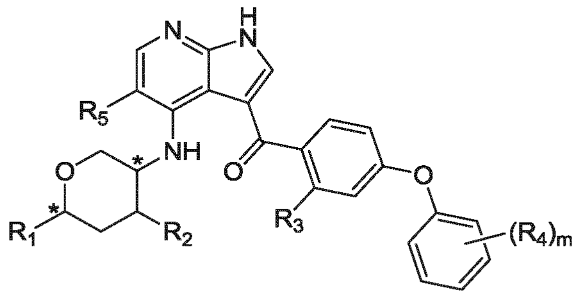

(I-1)

Wherein, $R_1$, $R_2$, $R_3$, $R_4$, $R_5$ and m are as defined in the present invention herein. The carbon atoms with "*" are chiral carbon atoms, and exist in the form of (*R*) or (S) single enantiomer or enriched in one enantiomer.

In some embodiments of the present disclosure, $R_1$ is selected from CN, $CH_3$, $CH_2CH_3$ and $CH(CH_3)_2$, wherein the $CH_3$, $CH_2CH_3$ and $CH(CH_3)_2$ are optionally substituted by 1, 2 or 3 $R_a$ and other variables are as defined herein.

In some embodiments of the present disclosure, $R_1$ is selected from CN and $CH_2OH$, and other variables are as defined herein.

In some embodiments of the present disclosure, $R_2$ is selected from H, $OCH_3$, $OCH_2CH_3$ and $OCH(CH_3)_2$, and other variables are as defined herein.

In some embodiments of the present disclosure, $R_3$ is Cl, and other variables are as defined herein.

In some embodiments of the present disclosure, $R_4$ is selected from H and F, and other variables are as defined herein.

In some embodiments of the present disclosure, the structural unit

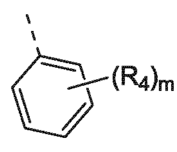

is selected from

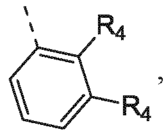

other variables are as defined herein.

In some embodiments of the present disclosure, the compound or pharmaceutically acceptable salt thereof is

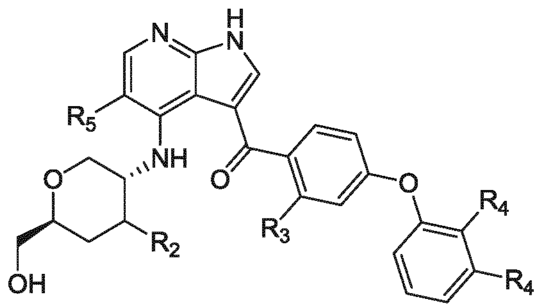

(I-2)

wherein, $R_1$, $R_2$, $R_3$, $R_4$ and $R_5$ are as defined herein.

The present disclosure also includes some embodiments that are obtained by combining any of the above-mentioned variables.

The present disclosure also provides the compound represented in the following formula or its pharmaceutically acceptable salt.

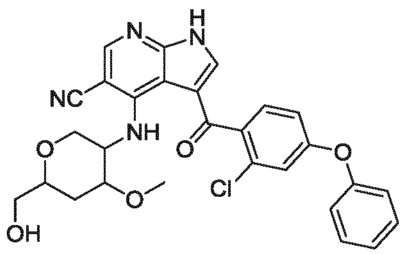

In some embodiments of the present disclosure, the compound or pharmaceutically acceptable salt thereof is

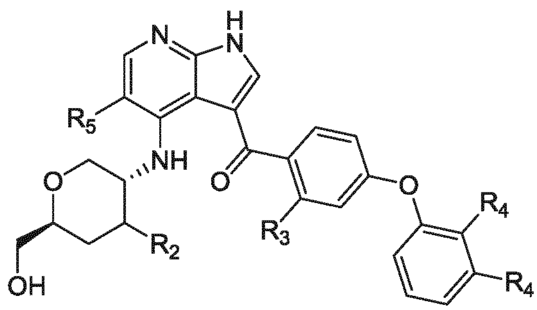

(I-2)

The present disclosure also provides the use of the above-mentioned compound or a pharmaceutically acceptable salt thereof in the manufacture of a medicament related to tumor.

The present disclosure also provides the use of the above-mentioned compound or a pharmaceutically acceptable salt thereof in the manufacture of a medicament related to hematomas.

The present disclosure also provides the following test methods:

**Assay example 1: BTK cell activity assay**

1. Cell culture and passage

Culture medium, trypsin and 1 × PBS used in the cell passage process were preheated in a 37°C water bath. The supernatant was removed.

1 mL trypsin was added to rinse, and then the rinse was removed. 1 mL of trypsin was added and digested at 37°C until the cells fell off. 10 mL culture solution was added and mixed well. The mixture was centrifuged at 1000 rpm for 5 min, and the cells were resuspended in 10 mL of culture medium. 0.7 mL of cell suspension was pipetted into a counting cup, and counted on ViCell XR to obtain a cell density is 0.327 million/mL, respectively. 1.17 mL of cell suspension and culture medium were weighed, respectively, to dilute the cell suspension. 100 μL phosphate buffer solution was added to the peripheral wells of the 384-well microplate, and 40 μL of cell suspension was added to other wells, respectively, and then the cell plate was cultured in an incubator.

2. Dosing

(1) Preparation of compound: A 9 μL dilution of the compound to be assayed was added to a shallow-well plate for Pod (Labcyte, #LP-0200). The shallow-well plate was centrifuged at 2500 rpm for 30 s.
(2)The cell plate was removed from the incubator.
(3) The compound was serially diluted 3-fold in Pod to obtained 10 concentrations, The diluted compound was added to the cell plate at 100 μL, respectively, and then the cell plate was returned to the incubator for culture.

3. 20 μL CellTier Glo was added to the plate of Day0 with DMSO added, and the mixture was shaken under the protection from light for 10 minutes. the plate was read with envision.

4. At Day 5: CTG was added and the plate was read.

(1) After being cultured for 72h, 20 μL Cell Tier Glo was added to the cell plate, and the mixture was shaken under the protection from light for 10 minutes.
(2) The plate was read with envision.

**Assay results**

1. Average value and standard deviation of 0% inhibition (DMSO well, MAX) and 100% inhibition (day0, DMSO) in each group was calculated;
2.

Inhibition rate (%) = (1 - (sample value-average value of 100% inhibition)/ (average value of 0% inhibition – average value of 100% inhibition)) * 100.

3. The curve is fitted by GraphPad 5.0 software.

**Assay example 2: pharmacokinetics study in vivo**

Assay example purpose: To assay the pharmacokinetics of compounds in CD-1 mice.

Assay method: The compounds to be assayed were mixed with vehicle. The mixture was vortexed and sonicated to obtain a clear solution. The candidate compounds solution was administrated intravenously to male CD-1 mice aged 7 to 10 weeks at a dose of about 0.2 mg/kg, or the candidate compound solution was given intragastrically at a dose of 1-3 mg/kg. The whole blood was collected for a certain period of time to prepare the plasma. The drug concentration was analyzed by LC-MS/MS. The pharmacokinetic parameters were calculated by Phoenix WinNonlin software (Pharsight Company, USA).

**Assay example 3: pharmacodynamics study in vivo**

Xenograft tumor model of TMD8 in SCID mouse:
Assay method:
A tumor model of subcutaneously transplanted human diffuse large B lymphoma in mice was established. Tumor cells in logarithmic growth phase were collected, counted, and then resuspended in RPMI1640. The cell suspension was adjusted to a concentration of $4 \times 10^7$/mL, and mixed well with Matrigel(1:1). Tumor cells were inoculated subcutaneously on the right back of the mouse, 4×106 cells/mouse. When the animal tumor reached about 100-300 $mm^3$, the tumor-bearing mice were randomly divided into groups according to the size of the tumor volume, with 6 mice in each group. On the day of the assay, the animals were administered the corresponding drugs according to the corresponding groups. The first group was set as a negative control group, which was administered 10%NMP/60%PEG400/30%H2O by gavage alone. The second group was set as a positive control group, which was given the assay compound at a dose of 30 mg/kg, once a day, for a total of 15 days.

During the assay, animal weight and tumor size were measured 3 times a week, and clinical symptoms of animals were observed and recorded every day. Each dosing was referenced to the most recent animal weight. For tumor measurement, length (a) and width (b) were measured with digital vernier calipers, and the calculation formula for tumor volume (TV) was: $TV=a \times b^2/2$.

**Definition and description**

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When compounds disclosed herein contain a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Unless otherwise specified, the term "isomer" is intended to include geometric isomers, cis- or trans- isomers, stereoisomers, enantiomers, optical isomers, diastereomers, and tautomers.

Compounds disclosed herein may be present in a specific geometric or stereoisomeric form. The present disclosure contemplates all such compounds, including cis and trans isomers, (-)- and (+)-enantiomers, (*R*)- and (*S*)-enantiomers, diastereoisomer, (*D*)-isomer, (*L*)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(+)" means dextroisomer, "(-)" means levoisomer, and "(±)" means racemate.

Unless otherwise specified, the wedge solid bond ( ) and a wedged dashed bond ( ) indicate the absolute configuration of a stereocenter; a straight solid bond ( ) and a straight dashed bond ( ) indicate the relative configuration of a stereocenter; a wavy line ( ) indicates a wedged solid bond ( ) or a wedged dashed bond( ), or a wavy line( ) indicates a straight solid bond ( ) and a straight dashed bond ( ).

Compounds disclosed herein may be present in a particular form. Unless otherwise specified, the terms "tautomer" or "tautomeric form" means that different functional groups are in dynamic equilibrium at room temperature and can be rapidly converted into each other. If tautomers are possible (as in solution), a chemical equilibrium of tautomers can be achieved. For example, proton tautomers (also known as prototropic tautomers) include interconversions by proton transfer, such as keto-enol isomerization and imine-enamine isomerization. Valence tautomers include interconversions by recombination of some bonding electrons. A specific example of keto-enol tautomerization is interconversion between two tautomers pentane-2, 4-dione and 4-hydroxypent-3-en-2-one.

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, 98% or more, 99% or more, 99.5% or more, 99.6% or more, 99.7% or more, 99.8% or more, or 99.9% or more.

Unless otherwise specified, the term "isomer excess" or "enantiomeric excess" means the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Optically active (*R*)- and (*S*)-isomer, or *D* and *L* isomer can be prepared using chiral synthesis or chiral reagents or other conventional techniques. If one kind of enantiomer of certain compound disclosed herein is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to afford the pure enantiomer. In addition, the enantiomer and the diastereoisomer are generally isolated through chromatography which uses a chiral stationary phase and optionally combines with a chemical derivative method (for example, carbamate generated from amine).

Compounds disclosed herein may contain an unnatural proportion of atomic isotopes at one or more of the atoms that make up the compounds. For example, a compound may be labeled with a radioisotope such as tritium ($^3H$), iodine-125 ($^{125}I$) or C-14 ($^{14}C$). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

Unless otherwise specified, the term "$C_{1-3}$ alkyl" refers to a linear or branched saturated hydrocarbon group containing 1 to 3 carbon atoms. The $C_{1-3}$ alkyl comprises $C_{1-2}$, $C_{2-3}$ alkyl and the like; It can be monovalent (such as methyl), divalent (such as methylene) or multivalent (such as methylene). Examples of $C_{1-3}$ alkyl include, but are not limited to, methyl (Me), ethyl (Et), propyl (including n-propyl and isopropyl), and the like.

Unless otherwise specified, the term "$C_{1-3}$ alkoxy" refers to an alkyl group containing 1 to 3 carbon atoms and attached to the remainder of a molecule through an oxygen atom. The $C_{1-3}$ alkoxy group includes $C_{1-2}$, $C_{2-3}$, $C_3$ and $C_2$ alkoxy, and the like. Examples of $C_{1-3}$ alkoxy include, but are not limited to, methoxy, ethoxy, propoxy (including n-propoxy and isopropoxy), and the like.

The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo. The term "optionally substituted" means an atom can be

substituted by a substituent or not, unless otherwise specified, the species and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to the embodiment disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuK$\alpha$ radiation in a scanning mode of $\phi/\omega$ scan; after collecting the relevant data, the crystal structure is further analyzed by the direct method (Shelxs97) to confirm the absolute configuration.

The term "protection group" includes, but is not limited to, "amino protection group", "hydroxyl protection group" or "sulfhydryl protection group". The term "amino protective group" refers to the protective group suitable for preventing the side reaction on the amino nitrogen position. Representative amino protection groups include, but are not limited to, formyl; Acyl, such as alkanoyl (for example acetyl, trichloroacetyl or trifluoroacetyl); Alkoxycarbonyl, such as tert-butoxycarbonyl (Boc); Aryl methoxycarbonyl, such as benzyloxycarbonyl (Cbz) and 9-fluorene methoxycarbonyl (Fmoc); Aryl methyl, such as benzyl (Bn), triphenylmethyl (Tr), 1,1-di - (4 '-methoxyphenyl) methyl; Methylsilyl, such as trimethylsilyl (TMS) and tert-butyldimethylsilyl (TBS), etc. The term "hydroxyl protection group" refers to the protection group suitable for preventing the hydroxyl from side reaction. Representative hydroxyl protection groups include, but are not limited to, alkyl, such as methyl, ethyl and tertiary butyl; Acyl, for example, alkanoyl (such as acetyl); Aryl methyl, such as benzyl (Bn), p-methoxybenzyl (PMB), 9-fluorenylmethyl (Fm) and diphenylmethyl (diphenylmethyl, DPM); Methylsilyl, such as trimethylsilyl (TMS) and tert-butyldimethylsilyl (TBS), and the like.

Solvents used in the present disclosure are commercially available.

Compounds are named according to general naming principles in the art or by ChemDraw® software, and commercially available compounds are named with their vendor directory names.

**Technical effect**

The compound of the invention has a strong inhibitory effect on $BTK^{C481S}$ mutation, has an inhibitory effect on tumor, and has good PK properties.

**Detailed description of the invention**

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

Intermediate A

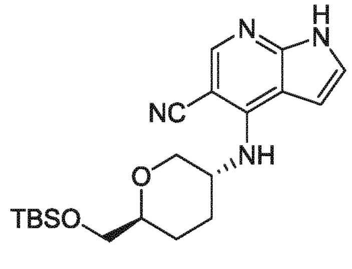

Route for synthesis:

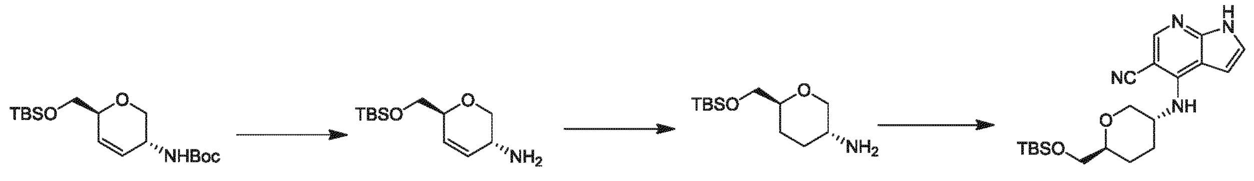

A1 A2 A3 A

Step 1: Synthesis of compound A2

To dissolve compound A1 (5 g, 14.55 mmol, 1 eq) in dichloromethane (5 mL), zinc bromide (13.11 g, 58.22 mmol, 2.91 mL, 4 eq) was added, and then the mixture was reacted at 20°C for 36 hours. After completion of the reaction, ammonia water (5 mL) was added into the reaction system, and saturated ammonium chloride aqueous solution (10 mL) was added. The organic phase was separated, and the aqueous phase was extracted with dichloromethane (20mL * 3). The organic phases were combined, dried with anhydrous sodium sulfate, and concentrated to give a crude intermediate A2. LCMS: (ESI)m/z: 244.2[M+1]$^+$.

Step 2: Synthesis of compound A3

Dry palladium carbon (0.3 g) was added to methanol (20 mL), and then compound A2 (2.5 g, 10.27 mmol, 1 eq) was added. The mixture was reacted under the condition of hydrogen atmosphere with pressure of 30 psi at 26°C for 24 hours. The mixture was filtered and concentrated to obtain compound A3. LCMS: (ESI)m/z: 246.2[M+1]$^+$.

Step 3: Synthesis of compound A

The compound of 4-chloro-1H-pyrrolo [2,3-B] pyridine-5-carbonitrile (810.41 mg, 4.56 mmol, 0.8 eq) and compound A3 (1.4 g, 5.70 mmol, 1 eq) were dissolved in N-methylpyrrolidone (2 mL), and N, N-diisopropylethylamine (1.84 g, 14.26 mmol, 2.48 mL, 2.5 eq) was added. The mixture was reacted in a microwave reactor at 200°C for 2 hours. 20 mL of water was poured into the reaction solution. then the mixture was extracted with ethyl acetate (20 mL * 4). The organic phase was combined, washed with 30 mL of saturated brine, and dried over anhydrous sodium sulfate to obtain a crude product. The crude product was purified by column chromatography (eluent: petroleum ether: ethyl acetate=30:1) to obtain compound A. LCMS: (ESI)m/z: 387.2[M+1]$^+$.

Example 1

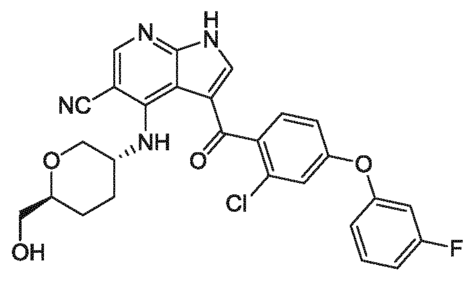

1

Synthesis route:

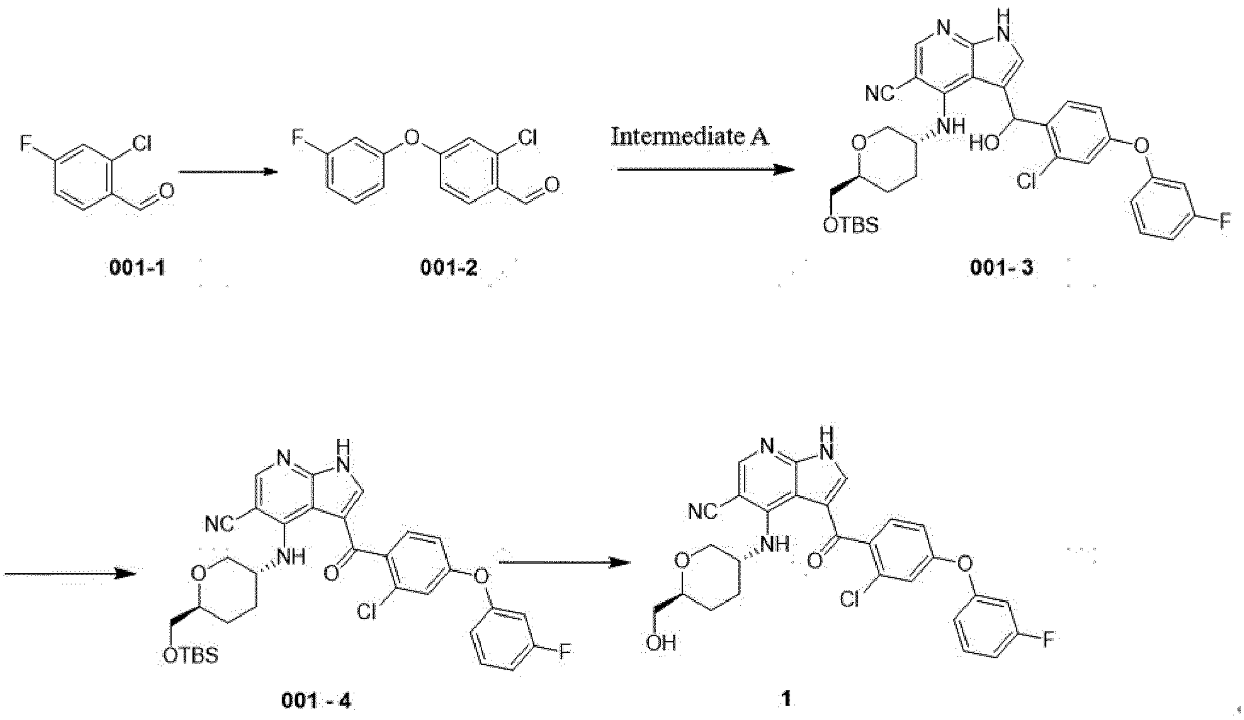


001-1 001-2 001-3

001-4 1

Step 1: Synthesis of compound 001-2

To dissolve 3-fluorophenol (5.30 g, 47.30 mmol, 4.35 mL, 1.5 eq), compound 001-1 (5.00 g, 31.53 mmol, 1 eq) in N, N-dimethylformamide (20 mL), cesium carbonate (15.41 g, 47.30 mmol, 1.5 eq) was added. The mixture was reacted at 80°C for 3 hours. The reaction solution was filtered, and 30 mL of water was added. Then the mixture was extracted with ethyl acetate (30 mL * 4). The organic phase was combined, and washed with 50 mL of saturated brine, dried over anhydrous sodium sulfate, and purified by column chromatography (eluent: petroleum ether: ethyl acetate=10:1) to obtain compound 001-2. $^1$H NMR(400MHz, $CDCl_3$) $\delta$ = 10.40 (s, 1H),7.95 (d, *J* = 8.8 Hz, 1H), 7.41 (dt, *J* = 6.8, 8.2 Hz, 1H), 7.07-6.96(m, 3H), 6.93-6.83(m, 2H).

Step 2: Synthesis of compound 001-3

Compound 001-2 (129.68 mg, 517.38 $\mu$mol, 1 eq) and intermediate A (0.2 g, 517.38 $\mu$mol, 1 eq) were added to methanol (5 mL), and then potassium hydroxide (145.15 mg, 2.59 mmol, 5 eq) was added. The mixture was reacted at 20°C for 72 hours. The reaction solution was concentrated and purified by column chromatography (eluent: dichloromethane: methanol=50:1) to obtain compound 001-3. LCMS:(ESI)m/z: 637.2[M+1]$^+$.

Step 3: Synthesis of compound 001-4

Compound 001-3 (0.13 g, 204.01 $\mu$mol, 1 eq) was added to dichloromethane (5 mL), and then manganese dioxide (532.09 mg, 6.12 mol, 30 eq) was added. the mixture was reacted at 30°C for 72 hours. After the completion of reaction, the solution was filtered and concentrated to obtain a crude product. The crude product was purified by column chromatography (eluent: dichloromethane: methanol=20:1) to obtain compound 001-4. LCMS:(ESI)m/z: 657.2[M+Na]+.

Step 4: Synthesis of compound 1

Compound 001-4 (0.12 g, 188.32 $\mu$mol, 1 eq) was added to tetrahydrofuran (3 mL), and then tetrabutylammonium fluoride (49.24 mg, 188.32 $\mu$mol, 1 eq) was added. The mixture was reacted at 20°C for 12 hours. The reaction solution was concentrated to obtain a crude product. The crude product is separated by preparative HPLC (chromatographic column: Welch Xtimate C18 150 * 25mm * 5 $\mu$m ; Mobile phase: [water (10mM $NH_4HCO_3$) - acetonitrile]; Acetonitrile%: 40% - 70%, 8 min) to obtain compound 1. LCMS:(ESI)m/z:521.1[M+1]$^+$. $^1$H NMR (400MHz,DMSO-$d_6$) $\delta$ = $^1$H NMR(400MHz,DMSO-$d_6$) $\delta$ = 9.65(br d, J = 8.4 Hz, 1H), 8.04 (s, 1H), 7.54-7.44 (m, 3H), 7.24 (d, J = 2.0 Hz, 1H), 7.13-6.97 (m, 4H), 4.72-4.68(m, 1H), 4.25-4.17(m, 2H), 2.36-2.25(m, 3H), 2.07-1.93(m, 1H), 1.82-1.69(m, 2H), 1.52-1.37(m, 2H).

Example 2

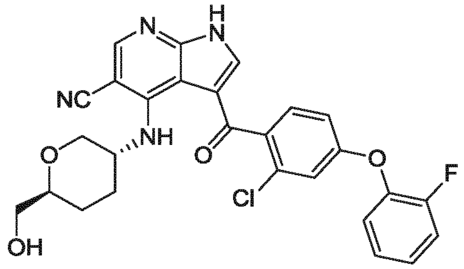

Synthesis route:

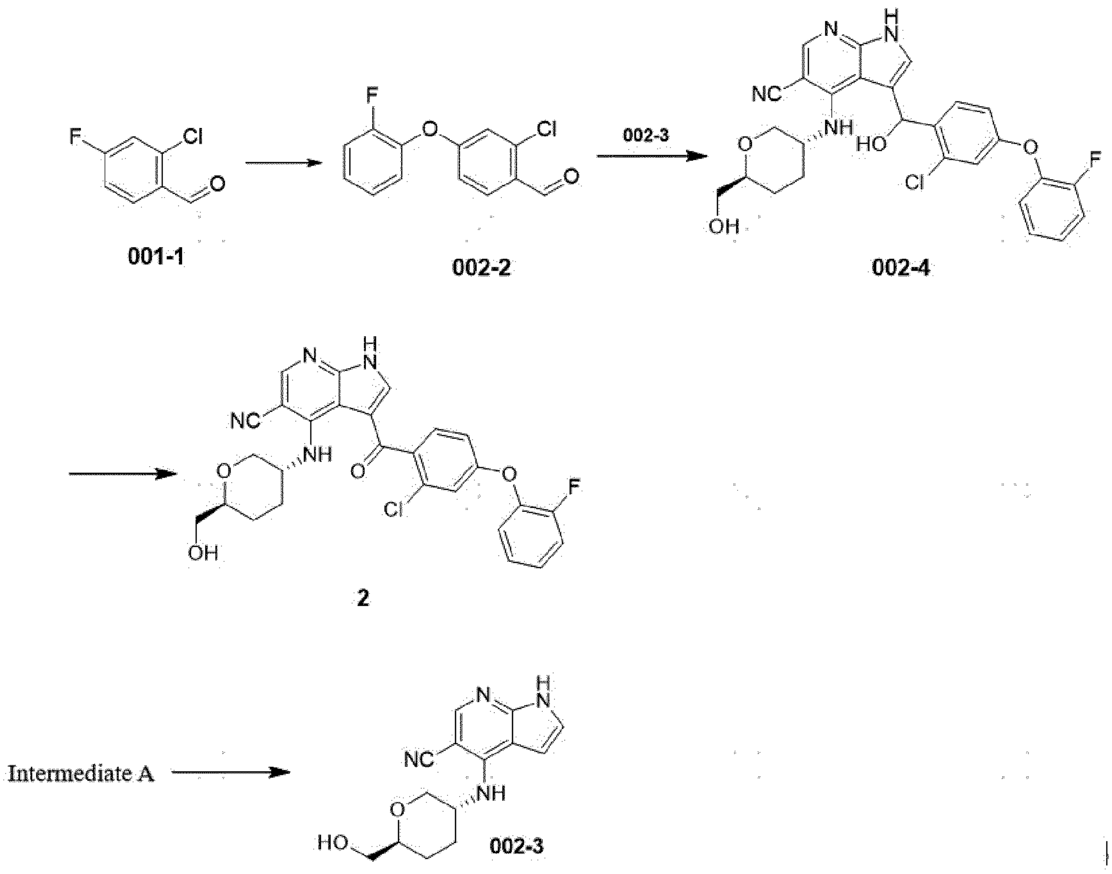


001-1

002-2

002-4

2

002-3

Step 1: Synthesis of compound 002-2

2-fluorophenol (5.30 g, 47.30 mmol, 4.38 mL, 1.5 eq) and compound 001-1 (5 g, 31.53 mmol, 1 eq) were dissolved in N, N-dimethylformamide (20 mL), and then cesium carbonate (15.41 g, 47.30 mmol, 1.5 eq) was added. The mixture was reacted at 80°C for 3 hours. The reaction solution was filtered, and 30 mL of water was added. The mixture was extracted with ethyl acetate (30 mL * 4), washed with 50 mL of saturated brine, dried over anhydrous sodium sulfate, and concentrated to obtain a crude product, The crude product was purified by column chromatography (eluent: petroleum ether: ethyl acetate=10:1) to obtain compound 002-2. $^1$H NMR(400MHz, $CDCl_3$) $\delta$ = 10.39 (s, 1H), 7.93(d, J = 8.5Hz, 1H), 7.28-7.13(m, 4H), 7.07-6.81(m, 2H).

Step 2: Synthesis of compound 002-3

to dissolve intermediate A (180 mg, 0.467 mmol, 1 eq) in dichloromethane (3 mL), hydrochloric acid/dioxane (4 M, 3 mL) was added. The mixture was reacted at 20°C for 3 hours, and then 20 mL of water was added. the reaction solution was extracted with dichloromethane (20 mL * 3). The organic phase was combined, dried over anhydrous sodium sulfate, filtered, and concentrated to obtain a crude product. The crude product was purified by column chromatography (eluent: dichloromethane: methanol=20:1) to obtain Compound 002-3. LCMS:(ESI) m/z:273.1$[M+1]^+$.

Step 3: Synthesis of compound 002-4

Compound 002-2 (441.84 mg, 1.76 mmol, 1.2 eq) and compound 002-3 (0.4 g, 1.47 mmol, 1 eq) obtained by step 2 were added to methanol (5 mL), and potassium hydroxide (412.08 mg, 7.34 mmol, 5 eq) was added. The mixture was reacted at 30 °C for 24 hours. The reaction solution was concentrated to obtain a crude product. The crude product was purified by column chromatography (eluent: dichloromethane: methanol=50:1) to obtain compound 002-4. LC-MS:(ESI) m/z:523.2$[M+1]^+$.

Step 4: Synthesis of compound 2

Compound 002-4 (164.14 mg, 313.87 μmol, 1 eq) was added to dichloromethane (10 mL), and manganese dioxide (818.63 mg, 9.42 mmol, 30 eq) was added. The mixture was reacted at 40°C for 5 hours. After the completion of the reaction, the mixture was filtered and concentrated to obtain a crude product. The crude product was purified by preparative HPLC (chromatographic column: Welch Xtimate C18 150 * 25mm * 5 μm ; Mobile phase: [water (10mM $NH_4HCO_3$) - acetonitrile]; Acetonitrile%: 40% - 70%, 8 min) to obtain compound 2. LCMS:(ESI)m/z:521.15$[M+1]^+$. $^1H$ NMR(400MHz, DMSO-$d_6$) δ = 9.65(br d, J = 8.4Hz, 1H), 8.04(s, 1H), 7.54-7.44(m, 3H), 7.24(d,J = 2.0Hz, 1H), 7.13-6.97(m,4H), 4.72-4.68(m, 1H), 4.25-4.17(m, 2H), 2.36-2.25(m, 3H), 2.07-1.93(m, 1H), 1.82-1.69(m, 2H), 1.52-1.37(m, 2H).

Example 3

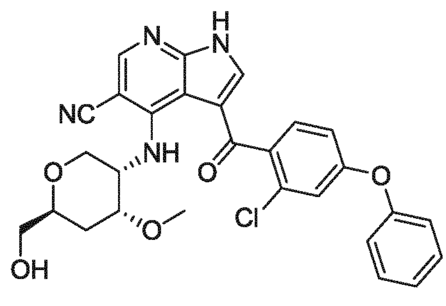

**3**

Synthesis route:

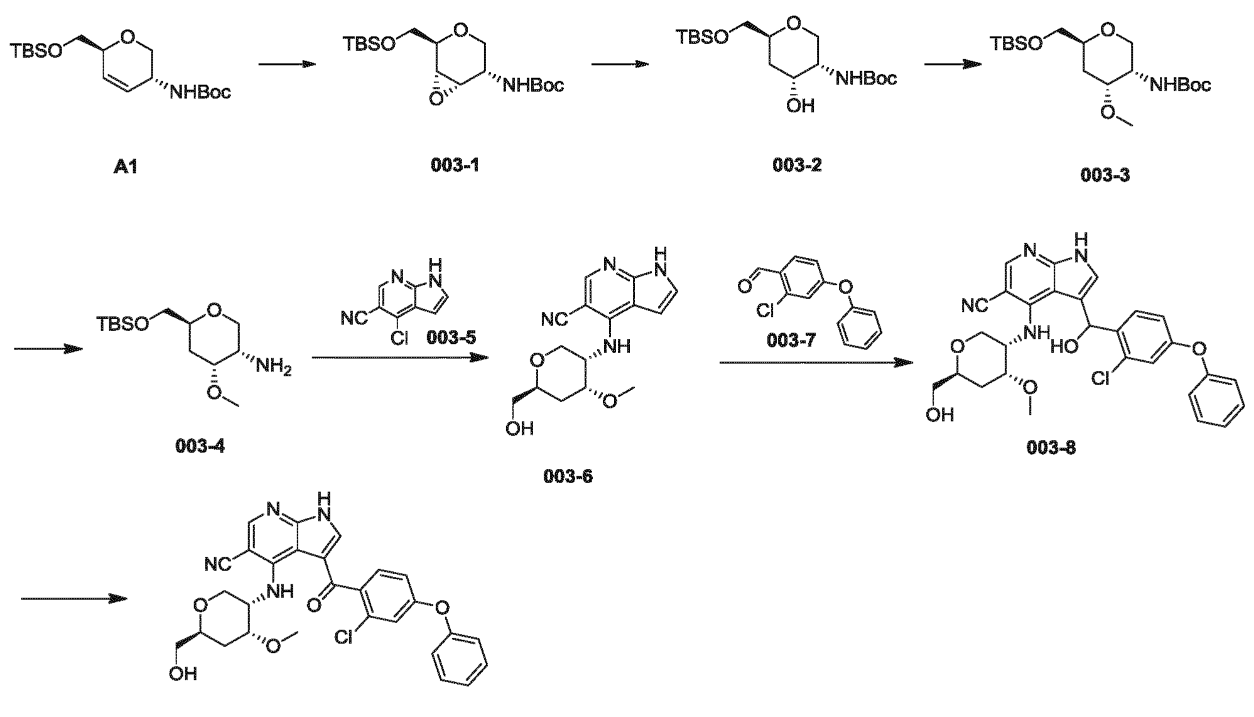
A1 003-1 003-2 003-3

003-4 003-5 003-6 003-7 003-8

3

Step 1: Synthesis of compound 003-1

To dissolve compound A1 (10 g, 29.11 mmol, 1 eq) in dichloromethane (100 mL), m-chloroperoxybenzoic acid (7.53 g, 43.66 mmol, 1.5 eq) was added at 0°C. the mixture was reacted at 20°C for 15 hours. The reaction solution was quenched by add saturated brine. The mixture was extracted with ethyl acetate (50 mL × 3), washed with saturated brine, dried over anhydrous sodium sulfate, and concentrated to obtain a crude product. The crude product was purified by column chromatography (eluent: petroleum ether: ethyl acetate=10:1) to obtain compound 003-1. LCMS:(ESI) m/z:304.2[M-$^t$Bu+1]$^+$.

Step 2: Synthesis of compound 003-2

To dissolve compound 003-1 (5 g, 13.91 mmol, 1 eq) in tetrahydrofuran (10 mL), lithium aluminum tetrahydrogen (2.5M, 8.34 mL, 1.5 eq) was added at - 20°C. the mixture was reacted at 20°C for 3 hours. After the completion of the reaction, the reaction was quenched by adding sodium hydroxide solution. The mixture was filtered. The filter cake was washed with ethyl acetate. The filtrate was combined, and concentrated to obtain a crude product. The crude product was purified by column chromatography (eluent: petroleum ether: ethyl acetate=3:1) to obtain compound 003-2. LC-MS:(ESI) m/z:306.1[M-$^t$Bu+1]$^+$.

Step 3: Synthesis of compound 003-3

To dissolve compound 003-2 (0.117 g, 323.61 μmol, 1 eq) in acetonitrile (2 mL), Silver oxide (224.97 mg, 970.83 μmol, 3 eq) and iodomethane (459.33 mg, 3.24 mmol, 201.46 μL, 10 eq) were added sequentially at 20°C under the atmosphere of nitrogen. Then the mixture was heated to 80°C for 12 hours. The reaction solution was cooled and then filtered, and the filtrate was concentrated to obtain compound 003-3. Compound 003-3 was directly used in the next step without purification. LCMS:(ESI) m/z:319.85[M-$^t$Bu+H]$^+$.

Step 4: Synthesis of compound 003-4

Combined compound 003-3 (700 mg, 1.86 mmol, 1 eq) obtained by parallel operation through step 3 was dissolved in dichloromethane (5 mL), and then zinc bromide (1.68 g, 7.46 mmol, 373.09 μL, 4 eq) was added. The mixture was reacted at 20°C for 48 hours, and then quenched with ammonia, extracted with dichloromethane (20 mL * 2).

Th eorganic phase was combined, dried over anhydrous sodium sulfate, filtered, and concentrated to obtain compound 003-4. Compound 003-4 was directly used in the next step without purification. LCMS:(ESI) m/z:276.1[M+1]$^+$.

Step 5: Synthesis of compound 003-6

Compound 003-5 (127.65 mg, 718.80 μmol, 0.6 eq), compound 003-4 (330 mg, 1.20 mmol, 1 eq), and N, N-diisopropylethylamine (464.50 mg, 3.59 mmol, 626.01 μL. 3 eq) were dissolved in N-methylpyrrolidone (2 mL). The mixture was reacted with microwave at 200°C for 2 hours. The reaction solution was poured into 20 mL of water, extracted with ethyl acetate (30 mL * 2). The organic phase was combined, dried over anhydrous sodium sulfate, concentrated, and separated by column chromatography (eluent: dichloromethane: methanol=50:1~20:1) to obtain 003-6. LCMS:(ESI) m/z:303.1$[M+1]^+$.

Step 6: Synthesis of compound 003-8

Compound 003-6 (70 mg, 231.54 μmol, 1 eq), compound 003-7(161.61 mg, 694.61 μmol, 3eq) were dissolved in methanol (3 mL), and then potassium hydroxide (38.97 mg, 694.61 μmol, 3 eq) was added. The mixture was reacted at 30°C for 72 hours, and then 30 mL of water was added, extracted with dichloromethane (30 mL * 3). The organic phase was combined, dried over anhydrous sodium sulfate, concentrated, and purified by column chromatography (eluent: dichloromethane: methanol=40:1) to obtained compound 003-8. LCMS:(ESI) m/z:535.1$[M+1]^+$.

Step 7: Synthesis of compound 3

to dissolved compound 003-8 (26 mg, 48.60 μmol, 1 eq) in dichloromethane (2 mL), manganese dioxide (169.01 mg, 1.94 mmol, 40 eq) was added. The mixture was reacted at 25°C for 72 hours, and then filtrated. The filtrate was concentrated and purified by preparative thin-layer chromatography (developing solvent: dichloromethane: methanol=20:1) to obtain compound 3. LCMS:(ESI) m/z:533.1$[M+1]^+$. $^1$H NMR(400MHz, $CDCl_3$)δ = 9.82(br d, J = 8.4Hz, 1H), 8.11(s,1H), 7.36-7.25(m,4H), 7.16(s,1H), 7.04-7.01(m, 3H), 6.87-6.90(m, 1H), 4.62(s, 1H), 4.05(s, 1H), 3.71-3.93(m, 3H), 4.60-4.44(m, 2H), 3.43(s, 3H), 1.94-1.93(m, 1H), 1.67-1.64(m, 1H).

Example 4

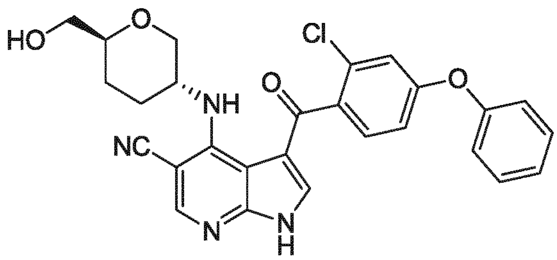

**4**

Synthesis route:

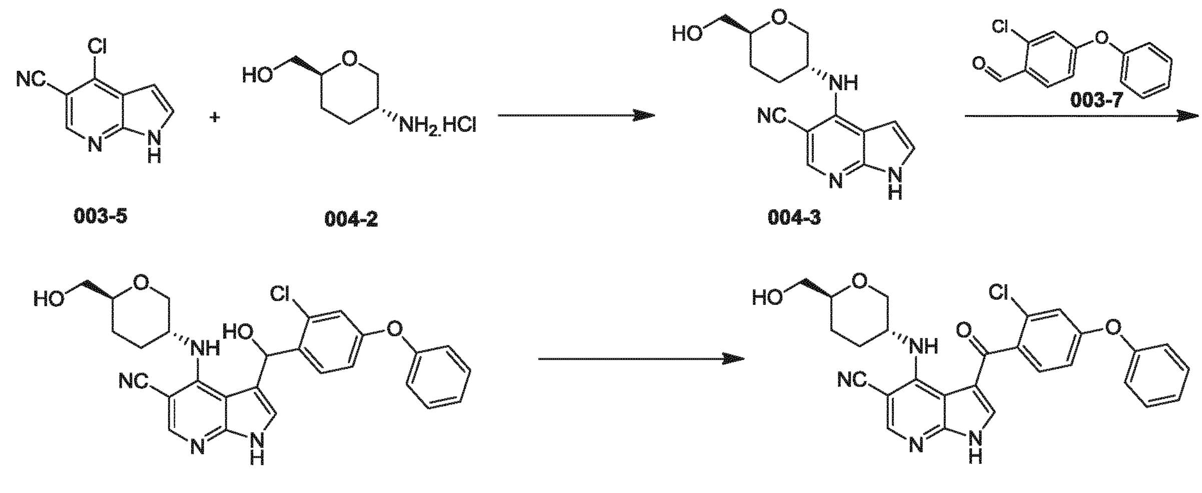


003-5 004-2 004-3

004-4 4

Step 1: Synthesis of compound 004-3

To dissolved compound 003-5 (0.1 g, 563.09 μmol, 1 eq) and compound 004-2(94.39 mg, 563.09 μmol, 1eq, HCl) in N-methylpyrrolidone (2 mL), N, N-diisopropylethylamine (145.55 mg, 1.13 mmol, 196.16 μL, 2 eq) was added. The mixture was reacted with microwave at 200°C for 4 hours at 200°C. The reaction solution was poured into 15 mL water, extracted with ethyl acetate (20 mL * 2). The organic phase was combined, washed with saturated brine (10 mL * 2), dried over anhydrous sodium sulfate, concentrated, and purified by column chromatography (eluent: dichloromethane: methanol=50:1 to dichloromethane: methanol=20:1) to obtain compound 004-3. LCMS:(ESI) m/z:273.2[M+1]$^+$. $^1$H NMR(400MHz,$CDCl_3$)δ = 11.13(br s, 1H), 7.98(s, 1H), 7.00(br s, 1H), 6.47(br d, *J* = 2.4Hz, 1H),4.97(br d, *J* = 8.4Hz, 1H), 4.16-4.03(m, 2H), 3.48-3.42(m, 2H), 3.25-3.10(m, 2H), 2.22-2.18(m, 2H), 1.91-1.85(m, 1H), 1.52-1.37(m, 2H).

Step 2: Synthesis of compound 004-4

The compounds 004-3 (0.3 g, 1.10 mmol, 1 eq) and compound 003-7 (281.96 mg, 1.21 mmol, 1.1 eq) obtained according to step 1 were dissolved in MeOH (5 mL), and KOH (154.53 mg, 2.75 mmol, 2.5 eq) was added. The mixture was reacted at 20°Cfor 36 hours. After the completion of the reaction, the reaction solution was poured into 50 mL water, extracted with dichloromethane (50 mL * 2). The organic phase was combined, dried over anhydrous sodium sulfate, concentrated and purified by preparative HPLC (chromatographic column: Phenomenex Luna C18 100 * 30 mm * 5 μm ; Mobile phase: [water (0.04% HCl) - acetonitrile]; Acetonitrile%: 30% - 60%, 10min) to obtain compound 004-4. LCMS:(ESI) m/z:505.3[M+1]$^+$.

Step 3: Synthesis of compound 4

To dissolved compound 004-4 (40 mg, 79.21 μmol, 1 eq) in DCM (10 mL), $MnO_2$ (68.87 mg, 792.14 μmol, 10 eq) was added, the mixture was reacted at 20°C for 36 hours, and then filtered. The filtrate was concentrated and purified by preparative HPLC (chromatographic column: Phenomenex Gemini-NX C18 75 * 30 mm * 3 μm ; Mobile phase: [water (10 mM $NH_4HCO_3$) - acetonitrile]; Acetonitrile%: 42% - 72%, 10.5 min) to obtain compound 4. LCMS: (ESI) m/z: 503.2 [M+1]$^+$. $^1$H NMR(400MHz, DMSO-*d*$_6$)δ = 9.49(d, J = 8Hz, 1H), 8.23(s, 1H), 7.74(s, 1H), 7.56(d, J = 8Hz, 1H), 7.47(t, J = 8Hz, 2H), 7.27-7.23(m, 1H), 7.19-7.17(m, 2H), 7.03-7.01(m, 1H), 4.71-4.68(m, 1H), 4.34-4.21(m, 2H), 3.25-3.19(m, 3H), 1.79-1.23(m, 4H).

Example 5

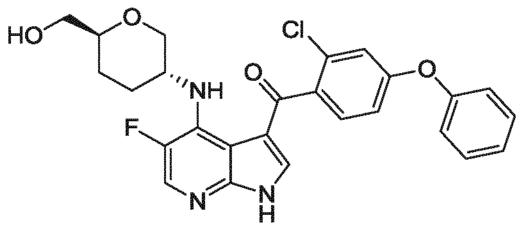

5

Synthesis route:

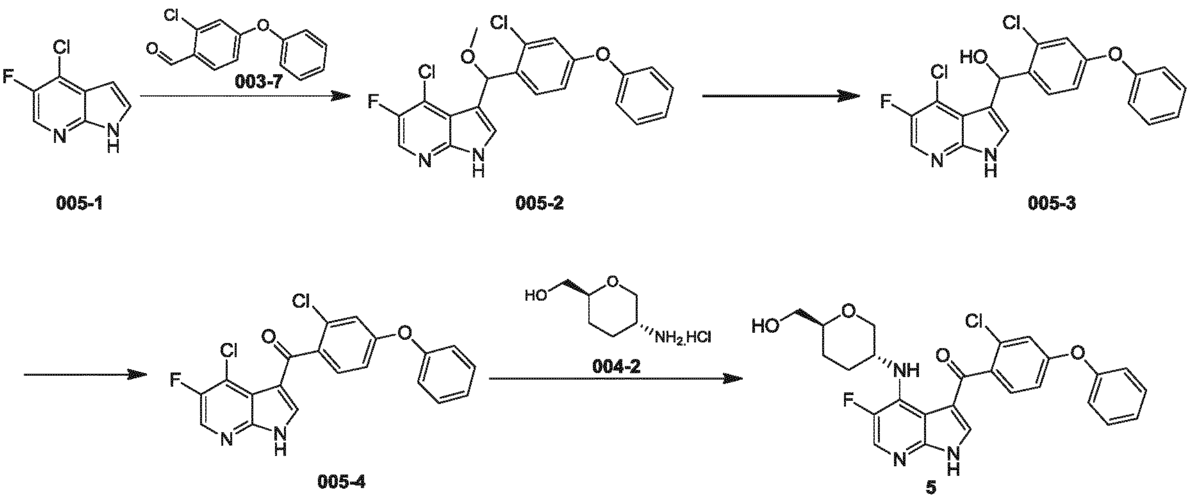

003-7

005-1

005-2

005-3

005-4

004-2

5

Step 1: Synthesis of compound 005-2

To dissolve compound 005-1 (980 mg, 5.75 mmol, 1 eq) and 003-7 (1.60 g, 6.89 mmol, 1.2 eq) in MeOH (25 mL), potassium hydroxide (1.13 g, 20.11 mmol, 3.5 eq) was added. The mixture was reacted at 25 °C for 36 hours. The reaction solution was poured into 50 mL water, adjusted the pH to 7 with 1N hydrochloric acid, and extracted with ethyl acetate (50ml * 2). The organic phase was combined, dried, concentrated over anhydrous sodium sulfate and purified by preparative HPLC (chromatographic column: Phenomenex Luna C18 100 * 30 mm * 5 μm ; Mobile phase: [water (0.04% HCl) - acetonitrile]; Acetonitrile%: 40% - 70%, 10 min.) to obtain compound 005-2. LCMS:(ESI) m/z:417.0[M+1]$^+$.

Step 2: Synthesis of compound 005-3

To dissolve compound 005-2 (0.3 g, 718.98 μmol, 1 eq) in acetic acid (2 mL), hydrobromic acid (745.00 mg, 4.42 mmol, 500.00 μL. 48% content, 6.15 eq) was added. The mixture was reacted at 25 °C for 20 minutes. The reaction solution was poured into 20 ml of water, adjusted the pH to 7 with saturated brine, extracted with dichloromethane (30 ml * 2). The organic phase was combined, dried over anhydrous sodium sulfate, filtered, concentrated to obtain compound 005-3. LCMS:(ESI) m/z:403.0[M+1]$^+$.

Step 3: Synthesis of compound 005-4

To dissolve compound 005-3 (0.28 g, 694.39 μmol, 1 eq) in DCM (20 mL), $MnO_2$ (845.15 mg, 9.72 mmol, 14 eq) was added. The mixture was reacted at 40°C for 16 hours, and then Filtered. The filtrate was concentrated and purified by column chromatography (eluent: dichloromethane: methanol=50:1 to dichloromethane: methanol=25:1) to obtain compound 005-4. LCMS:(ESI) m/z:401.0[M+1]$^+$.

Step 4: Synthesis of compound 5

To dissolve compound 005-4 (20 mg, 49.85 μmol, 1 eq) and compound 004-2(8.36 mg, 49.85 μmol, 1 eq, HCl)

in NMP (3 mL), N, N-diisopropylethylamine (12.89 mg, 99.70 μmol, 17.37 μL, 2 eq) was added. Then the mixture was reacted with microwave at 180°C for 4 hours. The reaction solution was poured into 10 mL of water, extracted with ethyl acetate (10 mL * 2). The organic phase was combined, washed with saturated brine (10 mL * 2), dried over anhydrous sodium sulfate, concentrated, and purified by preparative HPLC (chromatographic column: Phenomenex Luna C18 100 * 30 mm * 5 μm ; Mobile phase: [water (0.04% HCl) - acetonitrile]; Acetonitrile%: 30% - 60%, 10 min.) to obtain Compound 5. LCMS:(ESI) m/z:496.2$[M+1]^+$. $^1H$ NMR(400MHz, DMSO-$d_6$)δ = 8.57(br d, J = 7.6Hz, 1H), 8.08(d, J = 6.8Hz, 1H), 7.67(s, 1H), 7.56-7.46(m, 3H), 7.27-7.17(m, 4H), 7.03-7.01(m, 1H), 4.11-4.09(m, 1H), 3.81(br s, 1H), 3.59-3.52(m, 2H), 3.14(br t, J = 10.8Hz, 1H), 2.25-2.22(m, 1H), 1.78-1.75(m, 1H), 1.54-1.34(m, 2H).

**Biological assay data:**

Assay example 1: BTK enzyme activity assay

The specific assay process of BTK enzyme activity assay is as follows:
Buffer solution: 20 mM hydroxyethyl piperazine ethanesulfonic acid (Hepes) (pH 7.5), 10 mM magnesium chloride, 1 mM ethylenebis(oxyethylenenitrilo)tetraacetic acid (EGTA), 0.02% polyoxyethylene dodecyl ether (Brij35), 0.02 mg/mL BSA, 0.1mM sodium vanadate ($Na_3VO_4$), 2 mM dithiothreitol (DTT), 1% DMSO, 200 μM adenosine triphosphate (ATP).

1. The substrate was prepared in freshly prepared reaction buffer;
2. The required cofactor was added to the above-mentioned substrate solution;
3. The kinase $BTK^{C481S}$ was added to the above-mentioned substrate solution and mix well;
4. The compound dissolved in DMSO was added to the kinase reaction mixture through Echo550 (Acoustic technology; nanolite range), and the mixture was incubated at room temperature for 20 minutes;
5. $^{33}P$-ATP (specific activity of radioactivity is 10 μCi/μL) was added to the reaction mixture to initiate the reaction;
6. The mixture was incubated at room temperature for 2 hours;
7. The radioactivity was detected by filter-binding method;
8. Kinase activity data represented the percentage of remaining kinase activity in the assay sample compared to the vehicle (dimethyl sulfoxide) reaction. $IC_{50}$ value and fitted curve were obtained using Prism (GraphPad software), and the results were shown in Table 1.

Table 1. Results BTK activity assay in vitro

| Compound No. | $BTK^{C481S}$ $IC_{50}$(nM) |
|---|---|
| 1 | 58.9 |
| 2 | 11.7 |
| 3 | 74.2 |
| 4 | 7.4 |
| 5 | 8.9 |
| Conclusion: The compounds of the present disclosure have strong inhibitory effect on $BTK^{C481S}$ mutation. | |

## Claims

**1.** A compound represented by formula (P) or a pharmaceutically acceptable salt thereof,

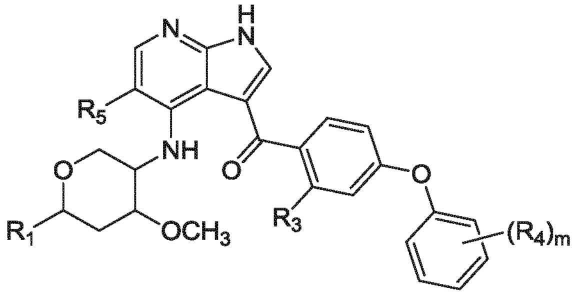

(P)

wherein,

$R_1$ is CN or $C_{1-3}$ alkyl, wherein $C_{1-3}$ alkyl is optionally substituted by 1, 2 or 3 $R_a$;
$R_3$ is selected from the group consisting of H, F, Cl, Br and I;
$R_4$ is selected from the group consisting of H, F, Cl, Br and I;
$R_5$ is CN or F;
m is 1 or 2;
$R_a$ is selected from the group consisting of H, F, Cl, Br and OH.

2. The compound or pharmaceutically acceptable salt thereof according to claim 1, which is

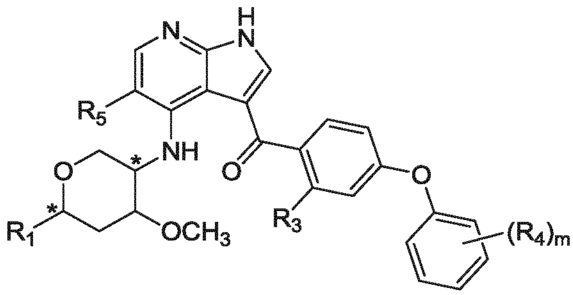

(P-1)

wherein, $R_1$, $R_3$, $R_4$, $R_5$ and m are as defined in claim 1;
the carbon atoms with "*" are chiral carbon atoms, wherein the compound is a single enantiomer in the form of (R) or (S) configuration or is a mixture of enantiomers enriched in one enantiomer.

3. The compound or pharmaceutically acceptable salt thereof according to claim 1 or 2, wherein, $R_1$ is selected from the group consisting of CN, $CH_3$, $CH_2CH_3$ and $CH(CH_3)_2$, wherein $CH_3$, $CH_2CH_3$ and $CH(CH_3)_2$ are optionally substituted by 1, 2 or 3 $R_a$.

4. The compound or pharmaceutically acceptable salt thereof according to claim 3, wherein $R_1$ is CN or $CH_2OH$.

5. The compound or pharmaceutically acceptable salt thereof according to claim 1 or 2, wherein $R_3$ is Cl.

6. The compound or pharmaceutically acceptable salt thereof according to claim 1 or 2, wherein $R_4$ is H or F.

7. The compound or pharmaceutically acceptable salt thereof according to claim 1 or 2, wherein, a structural unit of

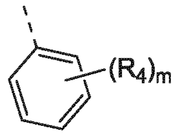

is

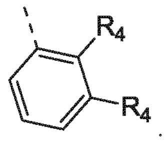

**8.** The compound or pharmaceutically acceptable salt thereof according to claim 1 or 2, wherein, the structural unit of

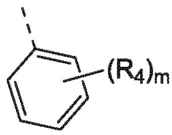

is selected from the group consisting of

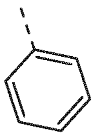

,

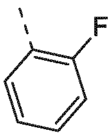

and

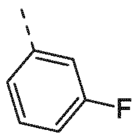

.

**9.** The compound or pharmaceutically acceptable salt thereof according to any one of claims 1 to 8, which is

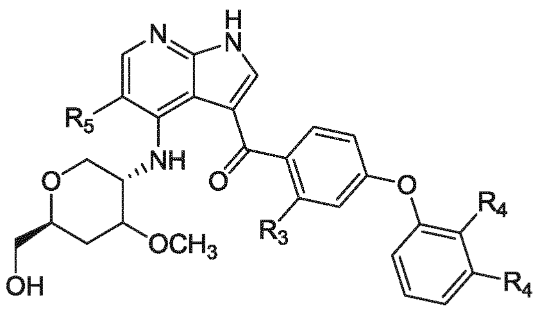

(P-2)

wherein,
$R_3$, $R_4$ and $R_5$ are as defined in any one of claims 1-8.

**10.** A compound as shown in the following formula or a pharmaceutically acceptable salt thereof

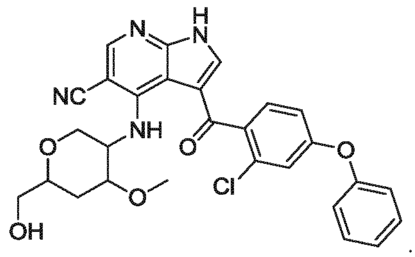

11. The compound or pharmaceutically acceptable salt thereof according to claim 10, which is:

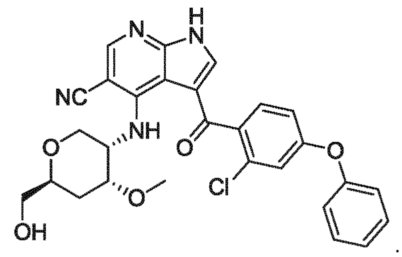

12. Use of the compound or pharmaceutically acceptable salt thereof according to any one of claims 1-11 in the manufacture of a medicament related to tumor.

## INTERNATIONAL SEARCH REPORT

International application No.
**PCT/CN2021/136117**

**A. CLASSIFICATION OF SUBJECT MATTER**

C07D 487/04(2006.01)i; C07D 471/04(2006.01)i; A61K 31/437(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D 487/- C07D471/- A61K 31/- A61P 35/-

Documentation searched other than minimum documentation to the extent that such documents are included in the fields searched

Electronic data base consulted during the international search (name of data base and, where practicable, search terms used)

CNPAT, WPI, EPODOC, CNKI, ISI Web of Knowledge, CAPLUS(STN), REGISTRY(STN): 南京明德新药研发有限公司, 张杨, 伍文韬, 耿开骏, 徐洋洋, 陈曙辉, 吡咯, 吡啶, BTK, 蛋白激酶, 癌, pyrrol+, pyrid+, protein kinase

**C. DOCUMENTS CONSIDERED TO BE RELEVANT**

| Category* | Citation of document, with indication, where appropriate, of the relevant passages | Relevant to claim No. |
|---|---|---|
| X | WO 2020239124 A1 (FOCHON PHARMACEUTICALS, LTD. et al.) 03 December 2020 (2020-12-03)<br>abstract, description, page 31, 4th-8th compounds, page 32, 8th, 11th-12th, 17th-18th compounds, page 33, 13th-14th compounds, page 34, 19th, 21st compounds, page 35, last compound, page 36, 1st, 11th compounds, page 38, 16th, 20th compounds, description, paragraph [10] | 1-12 |
| A | CN 101243084 A (PLEXXIKON, INC.) 13 August 2008 (2008-08-13)<br>description paragraphs [0006]-[008], [0093] | 1-12 |
| A | CN 105906631 A (PLEXXIKON, INC.) 31 August 2016 (2016-08-31)<br>description paragraphs [0006]-[0031], [0139]-[0167], [0998] | 1-12 |
| A | WO 2007002433 A1 (PLEXXIKON, INC. et al.) 04 January 2007 (2007-01-04)<br>description paragraphs [0008], [0291] | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 February 2022** | **09 March 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/136117**

| Patent document cited in search report | Publication date (day/month/year) | Patent family member(s) | Publication date (day/month/year) |
|---|---|---|---|
| WO 2020239124 A1 | 03 December 2020 | CA 3137985 A1 | 03 December 2020 |
| | | AU 2020283597 A1 | 25 November 2021 |
| CN 101243084 A | 13 August 2008 | US 2015290205 A1 | 15 October 2015 |
| | | RU 2008100933 A | 27 July 2009 |
| | | RU 2418800 C2 | 20 May 2011 |
| | | ES 2371397 T3 | 30 December 2011 |
| | | NO 2014006 I2 | 20 March 2014 |
| | | NO 2014006 I1 | 02 June 2014 |
| | | EP 1893612 A1 | 05 March 2008 |
| | | EP 1893612 B1 | 03 August 2011 |
| | | IL 188248 D0 | 13 April 2008 |
| | | IL 188248 A | 30 April 2012 |
| | | AT 518860 T | 15 August 2011 |
| | | RS 52010 B | 30 April 2012 |
| | | US 2016176865 A1 | 23 June 2016 |
| | | US 2010249118 A1 | 30 September 2010 |
| | | US 7863288 B2 | 04 January 2011 |
| | | CR 9677 A | 26 March 2008 |
| | | PE 20070100 A1 | 10 March 2007 |
| | | CY 1111996 T1 | 05 August 2015 |
| | | AR 078519 A2 | 16 November 2011 |
| | | US 2013303534 A1 | 14 November 2013 |
| | | PT 1893612 E | 21 November 2011 |
| | | EP 3088400 A1 | 02 November 2016 |
| | | SI 1893612 T1 | 30 March 2012 |
| | | PL 1893612 T3 | 31 January 2012 |
| | | DK 1893612 T3 | 21 November 2011 |
| | | NO 20076659 L | 18 March 2008 |
| | | NO 333913 B1 | 21 October 2013 |
| | | RU 2011101140 A | 20 July 2012 |
| | | RU 2565071 C2 | 20 October 2015 |
| | | MY 153898 A | 15 April 2015 |
| | | CA 2613015 A1 | 04 January 2007 |
| | | CA 2613015 C | 03 April 2012 |
| | | EP 2395004 A2 | 14 December 2011 |
| | | EP 2395004 A3 | 04 April 2012 |
| | | EP 2395004 B1 | 20 January 2016 |
| | | ES 2565992 T3 | 08 April 2016 |
| | | TW 200804370 A | 16 January 2008 |
| | | TW I432193 B | 01 April 2014 |
| | | AU 2006261993 A1 | 04 January 2007 |
| | | AU 2006261993 B2 | 17 November 2011 |
| | | KR 20080030619 A | 04 April 2008 |
| | | KR 101125919 B1 | 12 June 2012 |
| | | US 2013261117 A1 | 03 October 2013 |
| | | UA 95244 C2 | 25 July 2011 |
| | | TW 201345906 A | 16 November 2013 |
| | | TW I473808 B | 21 February 2015 |
| | | CR 20130216 A | 02 July 2013 |
| | | AR 054624 A1 | 04 July 2007 |

Form PCT/ISA/210 (patent family annex) (January 2015)

**INTERNATIONAL SEARCH REPORT**
**Information on patent family members**

International application No.
**PCT/CN2021/136117**

| Patent document cited in search report | Publication date (day/month/year) | Patent family member(s) | Publication date (day/month/year) |
|---|---|---|---|
| | | CN 102603581 A | 25 July 2012 |
| | | CN 102603581 B | 24 June 2015 |
| CN 105906631 A | 31 August 2016 | PE 20121335 A1 | 12 October 2012 |
| | | MX 2012005827 A | 19 June 2012 |
| | | SG 10201407718 W A | 29 January 2015 |
| | | EP 2501236 A1 | 26 September 2012 |
| | | EP 2501236 A4 | 15 May 2013 |
| | | EP 2501236 B1 | 29 March 2017 |
| | | AU 2016200485 A1 | 18 February 2016 |
| | | AU 2016200485 B2 | 28 September 2017 |
| | | ES 2627911 T3 | 01 August 2017 |
| | | AU 2010321883 A1 | 31 May 2012 |
| | | JP 2016185958 A | 27 October 2016 |
| | | JP 6194389 B2 | 06 September 2017 |
| | | CA 2781287 A1 | 26 May 2011 |
| | | CA 2781287 C | 31 July 2018 |
| | | BR 112012011792 A2 | 13 October 2015 |
| | | MY 156210 A | 29 January 2016 |
| | | NZ 599872 A | 29 August 2014 |
| | | US 2011152258 A1 | 23 June 2011 |
| | | US 8673928 B2 | 18 March 2014 |
| | | WO 2011063159 A1 | 26 May 2011 |
| | | CL 2012001303 A1 | 05 October 2012 |
| | | CN 102740698 A | 17 October 2012 |
| | | JP 2013511541 A | 04 April 2013 |
| | | US 2014288070 A1 | 25 September 2014 |
| | | US 9617267 B2 | 11 April 2017 |
| | | RU 2012125070 A | 27 December 2013 |
| | | MA 33811 B1 | 03 December 2012 |
| | | KR 20120097512 A | 04 September 2012 |
| | | CO 6551689 A2 | 31 October 2012 |
| | | IL 219567 D0 | 28 June 2012 |
| WO 2007002433 A1 | 04 January 2007 | US 2015290205 A1 | 15 October 2015 |
| | | RU 2008100933 A | 27 July 2009 |
| | | RU 2418800 C2 | 20 May 2011 |
| | | ES 2371397 T3 | 30 December 2011 |
| | | NO 2014006 I2 | 20 March 2014 |
| | | NO 2014006 I1 | 02 June 2014 |
| | | EP 1893612 A1 | 05 March 2008 |
| | | EP 1893612 B1 | 03 August 2011 |
| | | IL 188248 D0 | 13 April 2008 |
| | | IL 188248 A | 30 April 2012 |
| | | AT 518860 T | 15 August 2011 |
| | | RS 52010 B | 30 April 2012 |
| | | US 2016176865 A1 | 23 June 2016 |
| | | US 2010249118 A1 | 30 September 2010 |
| | | US 7863288 B2 | 04 January 2011 |
| | | CR 9677 A | 26 March 2008 |
| | | PE 20070100 A1 | 10 March 2007 |
| | | CY 1111996 T1 | 05 August 2015 |

Form PCT/ISA/210 (patent family annex) (January 2015)

**INTERNATIONAL SEARCH REPORT**
**Information on patent family members**

International application No.
**PCT/CN2021/136117**

| Patent document cited in search report | Publication date (day/month/year) | Patent family member(s) | | | Publication date (day/month/year) |
|---|---|---|---|---|---|
| | | AR | 078519 | A2 | 16 November 2011 |
| | | US | 2013303534 | A1 | 14 November 2013 |
| | | PT | 1893612 | E | 21 November 2011 |
| | | EP | 3088400 | A1 | 02 November 2016 |
| | | SI | 1893612 | T1 | 30 March 2012 |
| | | PL | 1893612 | T3 | 31 January 2012 |
| | | DK | 1893612 | T3 | 21 November 2011 |
| | | NO | 20076659 | L | 18 March 2008 |
| | | NO | 333913 | B1 | 21 October 2013 |
| | | RU | 2011101140 | A | 20 July 2012 |
| | | RU | 2565071 | C2 | 20 October 2015 |
| | | MY | 153898 | A | 15 April 2015 |
| | | CA | 2613015 | A1 | 04 January 2007 |
| | | CA | 2613015 | C | 03 April 2012 |
| | | EP | 2395004 | A2 | 14 December 2011 |
| | | EP | 2395004 | A3 | 04 April 2012 |
| | | EP | 2395004 | B1 | 20 January 2016 |
| | | ES | 2565992 | T3 | 08 April 2016 |
| | | TW | 200804370 | A | 16 January 2008 |
| | | TW | I432193 | B | 01 April 2014 |
| | | AU | 2006261993 | A1 | 04 January 2007 |
| | | AU | 2006261993 | B2 | 17 November 2011 |
| | | KR | 20080030619 | A | 04 April 2008 |
| | | KR | 101125919 | B1 | 12 June 2012 |
| | | US | 2013261117 | A1 | 03 October 2013 |
| | | UA | 95244 | C2 | 25 July 2011 |
| | | TW | 201345906 | A | 16 November 2013 |
| | | TW | I473808 | B | 21 February 2015 |
| | | CR | 20130216 | A | 02 July 2013 |
| | | AR | 054624 | A1 | 04 July 2007 |
| | | CN | 102603581 | A | 25 July 2012 |
| | | CN | 102603581 | B | 24 June 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202011437580